Europäisches Patentamt

European Patent Office

Office européen des brevets

(19)

(11) Veröffentlichungsnummer: **0 056 103**
**B1**

(12) # EUROPÄISCHE PATENTSCHRIFT

(45) Veröffentlichungstag der Patentschrift:
16.01.85

(21) Anmeldenummer: 81109366.5

(22) Anmeldetag: 30.10.81

(51) Int. Cl.⁴: **A 61 M 25/00**

(54) **Katheterbesteck.**

(30) Priorität: **10.01.81 DE 3100545**

(43) Veröffentlichungstag der Anmeldung:
**21.07.82 Patentblatt 82/29**

(45) Bekanntmachung des Hinweises auf die Patenterteilung:
**16.01.85 Patentblatt 85/3**

(84) Benannte Vertragsstaaten:
**AT BE CH DE FR GB IT LI LU NL SE**

(56) Entgegenhaltungen:
**DE - A - 2 305 640**
**DE - A - 2 855 502**
**DE - B - 2 104 226**
**GB - A - 1 304 231**
**NL - A - 7 610 957**

(73) Patentinhaber: **INTERMEDICAT GMBH,**
**Gerliswilstrasse 43, CH-6020 Emmenbrücke (CH)**

(72) Erfinder: **Schmidt, Klaus, Brandenburger Strasse 16,**
**D-3501 Ahnatal (DE)**

(74) Vertreter: **von Kreisler, Alek et al, Patentanwälte Von**
**Kreisler-Schönwald-Fues-Keller Selting-Werner**
**Deichmannhaus am Hauptbahnhof, D-5000 Köln 1 (DE)**

**Beschreibung**

Die Erfindung bezieht sich auf ein Katheterbesteck, bestehend aus einem Führungsstück mit einem Kanal zum Durchlass eines Katheterschlauches, in dessen Lumen sich eine drahtförmige, flexible Seele mit stumpfer Spitze erstreckt, wobei die Seele und der Katheterschlauch an ihrem patientenfernen Ende miteinander verbindbare Kupplungsteile tragen und an dem patientenfernen Ende des Kanals ein Schutzschlauch für den Katheterschlauch und die Seele befestigt ist.

Die perkutane Katheterisierung von Arterien und Venen, z.B. der Vena jugularis externa, ist wegen des gekrümmten Verlaufes des Gefässes schwierig und führt oft zu Fehllagen der Katheterspitze, d.h. diese liegt nicht regelrecht in der Vena cava, sondern ist in eine andere Vene, wie z.B. Vena jugularis interna oder Vena subclavia, ausgewichen.

Zur Verhinderung von Katheterfehllagen ist es bekannt, eine Gefässsonde aus Stahldraht als Führungsschiene zu verwenden. Dabei wird nach Gefässpunktion mittels einer Kanüle zunächst die Gefässsonde in Form eines dünnen, flexiblen Führungsdrahtes in das Gefässlumen eingeführt, und anschliessend wird nach Entfernen der Kanüle auf das distale Ende, d.h. das dem Anwender zugewandte freie extrakutane Ende der Gefässsonde ein Katheterschlauch aufgeschoben, der bei weiterer Längsverschiebung automatisch dem Verlauf der Gefässsonde folgt. Die Spitze des Katheterschlauches lässt sich auf diese Weise sicher in der Vena cava plazieren. Anschliessend wird die Gefässsonde aus dem plazierten Katheterschlauch herausgezogen und entfernt. In diesem Zusammenhang ist es bekannt, eine Gefässsonde mit gekrümmter flexibler Spitze zu verwenden, die den gekrümmten Verlauf der Vena jugularis externa problemlos passiert (GB-A-1 304 231). Dieses Vorgehen ist jedoch durch die aufeinanderfolgende Einführung der Gefässsonde und des Katheterschlauches sowie das Erfordernis des Auffädelns des Katheterschlauches auf die Gefässsonde umständlich und zeitraubend. Ausserdem ist die Gewährleistung absolut steriler Kautelen bei der Verlegung des Katheterschlauches schwierig, weil der Anwender direkt mit der in die Vene einzuführenden Gefässsonde und dem Aussenmantel des Katheterschlauches in Berührung kommt, wodurch erhöhte Kontaminationsgefahr mit bakterieller Infizierung besteht.

Zur Behebung dieses Mangels wurden Katheterbestecke der eingangs erwähnten Art geschaffen, bei denen die Gefässsonde als drahtförmige Seele von vornherein innerhalb des Katheterschlauches untergebracht ist, damit beide Teile gemeinsam gleichzeitig in das Gefäss eingeführt werden und es ist ein Schutzschlauch für beide Teile vorgesehen (DE-A-2 305 640; DE-U-7 837 983). Hierbei ist die Spitze der Seele zu der Spitze des Katheterschlauches ein Stück zurückgesetzt oder schliesst mit ihr bündig ab, so dass der Katheterschlauch zwar praktisch über seine ganze Länge stabilisiert ist, jedoch an seiner Spitze von der Seele nicht nach der Art einer Gefässsonde geführt wird.

Der Erfindung liegt die Aufgabe zugrunde, ein als geschlossenes, kontaminationsfreies System handhabbares Katheterbesteck der eingangs erwähnten Art so auszubilden, dass die drahtförmige, flexible Seele nicht nur als den dünnen Katheterschlauch versteifender Mandrin wirksam ist, sondern unter Ausnutzung der flexiblen Spitze der Gefässsonde die Spitze des Katheterschlauches in die angestrebte Position in der Vene oder Arterie bringt, so dass praktisch keine Lagekorrekturen des Katheterschlauches erforderlich sind.

Diese Aufgabe wird bei einem Katheterbesteck der eingangs genannten Art dadurch gelöst, dass erfindungsgemäss das patientennahe Ende der Seele über die Spitze des Katheterschlauches ein Stück übersteht und dass das überstehende Stück der Seele sich in dem Kanal des Führungsstückes befindet.

Auf diese Weise wird die einfach zu handhabende Einheit aus Katheterschlauch und in diesem steckender drahtförmiger Seele in einem geschlossenen System mit einem Führungskopf versehen, der beim Vorschieben der Einheit in ein Gefässlumen als Sonde wirksam ist, die Engstellen und Winkel in dem Gefäss überwindet, ohne steckenzubleiben oder abgelenkt zu werden und sicher in die vorgeschriebene Position gelangt. Die Spitze des Katheterschlauches wird entsprechend korrekt geführt und plaziert und liegt nach Herausziehen der Seele regelrecht in der gewünschten Position. Nachträgliche Korrekturen von Fehllagen und eine möglicherweise damit verbundene Mehrfachpunktion des Gefässes wird vermieden und das Risiko einer Katheterisierung wird für den Patienten herabgesetzt. Die Verwendung des Katheterbesteckes mit einem Führungsstück mit Schutzschlauch hat den Vorteil, dass die Einführung der Seele und des Katheterschlauches kontaminationsfrei und steril auch unter Notfallbedingungen möglich ist und eine direkte Berührung durch den Anwender ausgeschlossen wird, so dass Kontaminationsgefahr mit bakterieller Infizierung unterbunden wird.

Die erwähnten Vorteile lassen sich dadurch noch vergrössern, dass das über die Spitze des Katheterschlauches überstehende Stück der Seele weich und flexibel ausgebildet, etwa halbkreisförmig gekrümmt und etwa so lang wie der Krümmungsabschnitt ist. Durch diese J-förmige Spitze der Seele erhält sie besonders günstige Führungseigenschaften, die eine Passage über Krümmungen und Venenklappen bis zur gewünschten Position erleichtern. Vorteilhafterweise ist das über die Spitze des Katheterschlauches überstehende Stück der Seele etwa 1,5 bis 3 cm lang, damit sie unmittelbar nach Austritt aus einer in das Gefäss eingesetzten Kapillare durch Memoryeffekt ihre vorgegebene J-Form annimmt und ihre optimale Führungseigenschaft erhält. Alternativ ist eine gerade Gestaltung des überstehenden Stückes der Seele möglich.

Es sind zwei Varianten der Erfindung vorteilhaft. Die Seele kann um das über die Spitze des Katheterschlauches überstehende Stück länger sein als der Katheterschlauch. In diesem Falle ist das mit dem extrakutanen, patientenfernen Ende der Seele durch Verkleben, Verschweissen usw. verbundene Kupplungsteil mit Luer-Konus mit einem Katheteransatz verriegelbar verbunden, wodurch der vorgegebene Überstand der J-Spitze der Seele über die Spitze des Katheterschlauches fixiert ist. Beide Teile werden gemeinsam in das Blutgefäss vorgeschoben, wobei der weiche und flexible Überstand der Seele die Führung übernimmt. Ferner ist es möglich, dass die Seele mit dem über die Spitze des Katheterschlauches überstehenden Stück etwa doppelt so lang ist wie der Katheterschlauch. Dabei steht nicht nur die Spitze der Seele über die Spitze des Katheterschlauches über, sondern ihr anderes extrakutanes Ende hat einen erheblichen Überstand zu dem entsprechenden Ende des Katheterschlauches. Hierbei sind die Kupplungsteile von Seele und Katheterschlauch zunächst nicht miteinander verbunden, und die Seele wird zuerst durch den festgehaltenen Katheterschlauch und die Kapillare in die Vene eingeschoben. Nachdem sie in der richtigen Tiefe eingeführt ist, was z.B. durch Markierungen kenntlich gemacht werden kann, wird der Katheterschlauch über die als Führung dienende Seele in die Vene geschoben.

Der Kanal des Führungsstückes kann als schräg in einen geraden Hauptkanal für eine Punktionskanüle mündender Seitenkanal nur für den Katheterschlauch bestimmt sein (DE-U-7 837 983) oder er kann ein gerader einziger Kanal sein, der sowohl die Punktionskanüle als auch den Katheterschlauch mit Seele durchlässt (DE-A-2 305 640). In beiden Fällen ist vorteilhafterweise der Kanal des Führungsstückes länger als das über die Spitze des Katheterschlauches überstehende Stück der Seele.

Bei Vorschieben der Einheit von Seele und Katheterschlauch innerhalb der Vene übernimmt die J-Spitze der Seele die Führung über Krümmungen und Venenklappen, während die Seele selbst zur Versteifung des hochflexiblen Katheterschlauches dient. Dadurch wird eine sichere Plazierung der Spitze des Katheterschlauches in der Vena cava garantiert. Nach Erreichen der vorgeschriebenen Position einige cm vor dem rechten Vorhof des Herzens wird unter Beibehaltung der Katheterposition nach vorhergehendem Entfernen des Führungsstückes durch Längsteilung desselben und nach Abnahme des Schutzschlauches zuerst die Kapillare aus der Vene herausgezogen und durch Längsteilung vom Katheterschlauch entfernt, danach wird die Seele aus dem Katheterschlauchlumen herausgezogen und eine Infusionsleitung eines Überleitungsgerätes am Katheteransatz angeschlossen.

In der Zeichnung sind Ausführungsbeispiel der Erfindung schematisch dargestellt. Es zeigen:

Fig. 1 eine Draufsicht einer Ausführungsform eines Katheterbesteckes mit Führungsstück,

Fig. 2 eine Draufsicht einer anderen Ausführungsform eines Katheterbesteckes mit Führungsstück und

Fig. 3 bis 5 Ausgestaltungen von für das Katheterbesteck geeigneten Führungsstücken in Draufsicht.

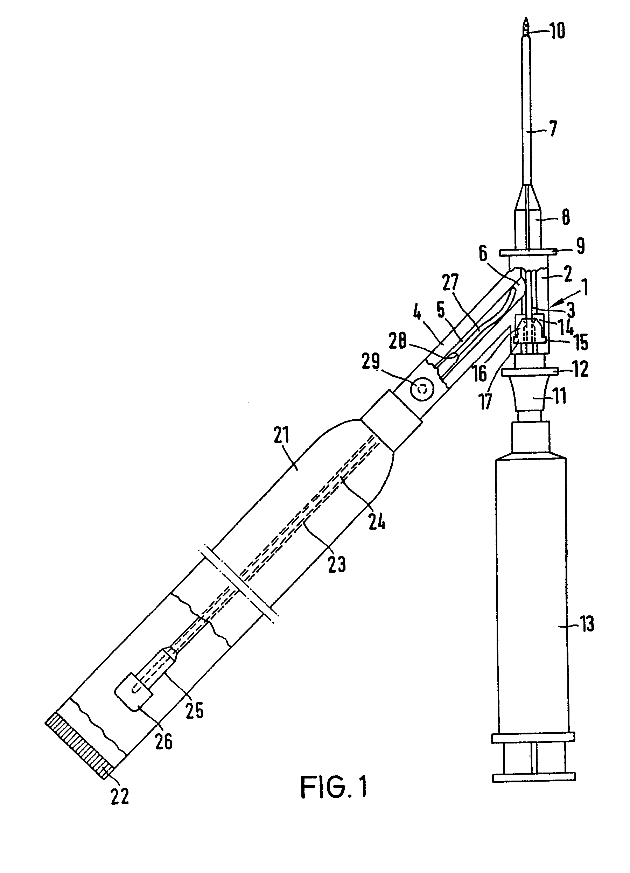

Gemäss Fig. 1 besteht ein Y-förmiges Führungsstück 1 aus einem geraden Stutzen 2, in dem ein gerader Hauptkanal 3 ausgebildet ist und an den unter einem Winkel ein schräger Stutzen 4 mit einem Seitenkanal 5 angeschlossen ist. Der Seitenkanal 5 mündet bei 6 in den Hauptkanal 3. An das dem Patienten zugewandte Ende des Stutzens 2 ist eine längsteilbare Kapillare 7 mit einem Ansatz 8 lösbar angeschlossen, der mit einer Griffplatte 9 versehen ist. Durch den Stutzen 2, den Ansatz 8 und die Kapillare 7 ragt eine Stahlkanüle 10 hindurch, deren angeschliffene Spitze über die Kapillare 7 übersteht und zur Punktion eines Gefässes, z.B. einer Vene oder Arterie, dient. Die Stahlkanüle 10 ist an einem Ansatz 11 befestigt, der eine Griffplatte 12 trägt und auf den Stutzen 2 abnehmbar aufgesteckt ist. Mit dem anderen Ende des Ansatzes 11 ist eine Spritze 13 verbunden.

Vor der Einmündung des Seitenkanals 5 ist der Hauptkanal 3 mit einer zylindrischen Erweiterung 14 versehen, die an ihrem der Spritze 13 zugewandten Ende eine Ringaussparung 15 aufweist. In der zylindrischen Erweiterung 14 ist ein Ventil untergebracht, dessen Ventilkörper 16 ein hutförmiger gummielastischer Teil mit geschlitztem Boden ist. Zur verschiebungssicheren Halterung des Ventilkörpers 16 dient ein Ringbund 17, der in die Ringaussparung 15 eingreift. Die Stahlkanüle 10 öffnet den Schlitz im Boden des Ventilkörpers 16. Nach ihrer Herausziehung aus dem Ventilkörper schliesst sich sein Schlitz automatisch sofort.

Ein weiteres Ausführungsbeispiel eines Ventilkörpers ist in Fig. 5 veranschaulicht. Ein gummielastischer Ventilkörper 18 ist in diesem Falle becherförmig gestaltet und der Boden des becherförmigen Teiles geht über eine Lochöffnung 19 in einen flexiblen Schlauchansatz 20 über, dessen Durchlass von einer Stahlkanüle aufweitbar ist und sich nach Herausziehen der Stahlkanüle sofort wieder bis zum Verschluss der Öffnung 19 radial zusammenzieht.

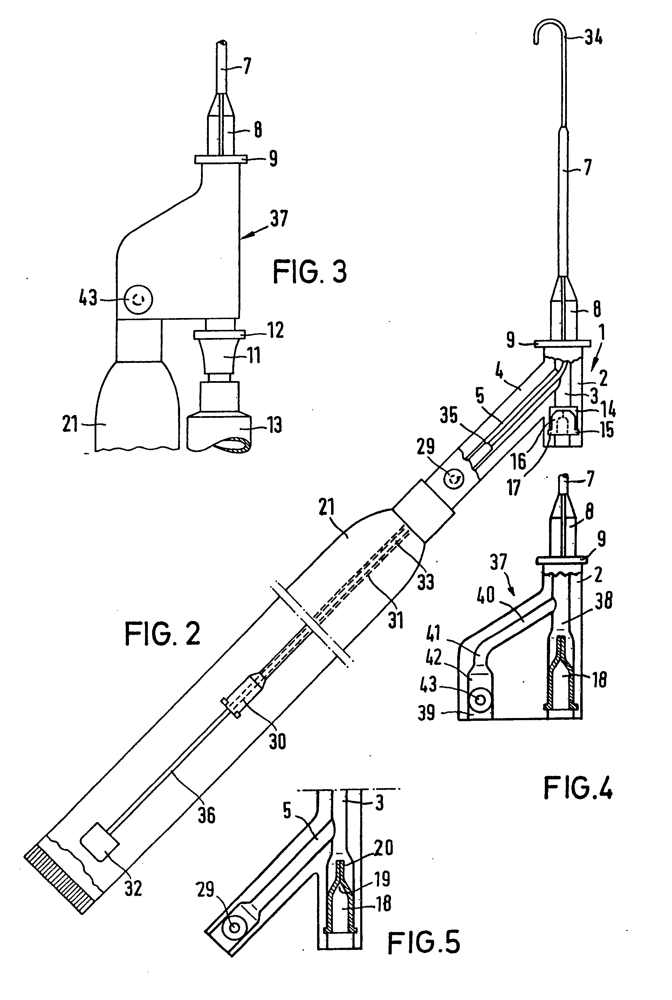

An dem äusseren Ende des schrägen Stutzens 4 ist ein durchsichtiger Folienhüllschlauch als Schutzschlauch 21 abnehmbar befestigt. Das Ende des Schutzschlauches 21 ist mittels einer Naht 22 verschlossen. Der Schutzschlauch 21 enthält ein Katheterbesteck, das aus einem dünnen flexiblen Katheterschlauch 23 und einer in diesem steckenden Seele 24 besteht. Der Katheterschlauch 23 ist an seinem extrakutanen Ende mit einem Katheteransatz 25 verbunden, der mit einem Kupplungsteil 26 am Ende der Seele 24 lösbar verriegelt ist. In diesem Zustand steht ein Stück 27 der Spitze der Seele 24 über die Spitze 28 des Katheterschlauches 23 über. Dieser Überstand 27 ist weich und flexibel ausgebildet und vorteilhaft mit einer Krümmungstendenz ausgestattet, die die Spitze der Seele 24 in freiem Zustand J-förmig krümmt (Fig. 2). Als Seele 24 kann eine Seldinger-Spirale mit 3 mm Krümmungsradius verwendet werden. Solange die Spitze der Seele 24 die in Fig. 1 gezeigte Position innerhalb des Seitenkanals 5 innehat, wird sie im wesentlichen gestreckt gehalten. Zusätzlich zu dem Überstand 27 befindet sich ein Stück des Spitzenabschnittes des Katheterschlauches 23 in dem Seitenkanal 5, der länger ist als der Überstand 27. Der Überstand 27 ist vorteilhaft ca. 2 cm lang. Die restliche Länge der Seele 24 entspricht derjenigen des Katheterschlauches 23. Dies bedingt, dass beide gemeinsam durch den Seitenkanal 5 in den Hauptkanal 3 und die Kapillare 7 eingeschoben werden, sobald die Stahlkanüle 10 herausgezogen worden ist. Nach Austritt des Überstandes 27 der Seele aus der Kapillare 7 nimmt er durch Memoryeffekt innerhalb der Vene sofort die vorgegebene J-Form an und führt den durch die Seele 24 versteiften hochflexiblen Katheterschlauch 23 über Krümmungen und Venenklappen bis zur sicheren Plazierung der Katheterspitze 28 in der Vena cava. Dieses Verlegen des Katheterschlauches erfolgt aufgrund des Führungsstückes 1 und des Schutzschlauches 21 kontaminationsfrei und steril auch unter Notfallbedingungen, weil eine direkte Berührung des Katheterschlauches 23 durch den Anwender entfällt. Zur Ermöglichung eines wechselweisen Vorschiebens des Katheterschlauches 23 mit Seele 24 und Strecken des Schutzschlauches dienen zwei Druckknopf-Bremsstücke, die am Stutzen 4 des Führungsstückes 1 einander gegenüberliegend angeordnet sind und von denen das Druckknopf-Bremsstück 29 sichtbar ist. Durch Zusammendrücken der Druckknopf-Bremsstücke 29 zwischen Daumen und Zeigefinger wird ein Herausgleiten des versteiften Katheterschlauches 23 aus der Vene beim Strecken des Schutzschlauches 21 verhindert.

Nach regelrechter Einführtiefe, die an Kathetermarkierungen ersichtlich sein kann, wird der Schutzschlauch 21 vom Stutzen 4 entfernt, und das Führungsstück 1 von dem Ansatz 8 und — durch Trennung seiner beiden Längshälften — vom Katheterschlauch abgenommen. Die Kapillare 7 wird sodann aus dem Stichkanal herausgezogen und durch Aufsplitten vom Katheterschlauch entfernt. Nach Röntgenkontrolle wird die Seele 24 aus dem Katheterschlauch 23 herausgezogen und eine vorbereitete Infusion angeschlossen. Der Katheter wird in seiner richtigen Position mit einem Fixierungsclip versehen und durch Nahtfixierung an der Punktionsstelle gesichert. Die weitere Fixierung des Katheters erfolgt in der herkömmlichen Technik mit einem Pflaster auf der Haut.

Die in Fig. 2 gezeigte Anordnung entspricht im wesentlichen derjenigen gemäss Fig. 1 mit der Ausnahme, dass der Ansatz 30 eines Katheterschlauches 31 nicht mit einem Kupplungsteil 32 einer in dem Katheterschlauch 31 steckenden Seele 32 verbunden ist. Die Seele 33 ist etwa doppelt so lang wie der Katheterschlauch 31. Ihre vorzugsweise mit 3 mm Krümmungsradius versehene Spitze 34 ragt als Überstand über die Spitze 35 des Katheterschlauches 31 in den Seitenkanal 5 des Führungsstückes 1 hinein. Ihre distale Überlänge 36 steht über den Ansatz 30 des Katheterschlauches 31 vor und ist wie der Katheterschlauch 31 insgesamt von dem sterilen Schutzschlauch 21 umgeben.

Mit diesem geschlossenen Kathetersystem mit einem Katheterschlauch 31, über dessen Spitze 35 die J-förmige Spitze 34 der Seele 33 ein Stück vorsteht, wobei die beiden Spitzen in dem Seitenkanal 5 eines Führungsstückes 1 geführt sind, wird so gearbeitet, dass die Seele 33 kontaminationsfrei durch den Seitenkanal 5 und die Kapillare 7 in das punktierte Blutgefäss eingeführt wird, indem der Katheterschlauch 31 mit der linken Hand durch Betätigung der beiden Druckknopf-Bremsstücke 29 in seiner Position gehalten wird, und die Seele 33 mittels des Kupplungsteiles 32 mit dem Schutzschlauch 21 durch stopfende Bewegung durch den Katheterschlauch 31 hindurchgeschoben wird. Nachdem die Seele 33 in der richtigen Tiefe eingeführt ist, was durch Markierungen kenntlich gemacht werden kann, wird der Katheterschlauch 31 mit dem Schutzschlauch 21 über die als Führung dienende Seele 33 in die Vene geschoben. Dabei betätigt die linke Hand zur Ermöglichung der Streckung des Schutzschlauches 21 wieder die Druckknopf-Bremsstücke 29, wobei der Bremsdruck ausreicht, um auch die Seele 33 gegen Herausrutschen zu sichern. Bei weiterem Vorschieben des Katheterschlauches 31 bis zum erneuten Strecken des Schutzschlauches 21 wird die Seele an ihrem extrakutanen Ende gegen eine Lageveränderung mit der zweiten Hand gesichert. Das weitere Vorgehen entspricht der zu dem Beispiel der Fig. 1 beschriebenen Handhabung, bei der sämtliche Systemteile durch Herausziehen bzw. Längsteilung vom Katheterschlauch 31 entfernt werden und dieser an eine Infusionsvorrichtung angeschlossen wird.

Fig. 3 zeigt ein Führungsstück 37 im kompakter Bauweise, bei dem der Hauptkanal 38 und der schräge Seitenkanal 39 wie in Fig. 4 dargestellt verlaufen. In dem geraden Hauptkanal 38 ist der in Fig. 5 gezeigte gummielastische Ventilkörper 18 angeordnet. Bei entsprechend anderer Ausbildung der seiner Aufnahme dienenden Erweiterung des Hauptkanals 38 kann jedoch auch der Ventilkörper 16 gemäss Fig. 1 und 2 verwendet werden. In den Hauptkanal 38 mündet hinter dem Ventilkörper 18 ein schräger Abschnitt 40 des Seitenkanals 39, an den sich ein gekrümmter Abschnitt 41 anschliesst, der in einen zum Hauptkanal 38 parallelen Abschnitt 42 übergeht. In dem Abschnitt 42 sind Druckknopf-Bremsstücke 43 vorgesehen und mit seinem Ende ist ein Schutzschlauch 21 abnehmbar verbunden. Auch mit diesem Führungsstück 37 kann sowohl das Katheterbesteck 23, 24 nach Fig. 1 als auch das Katheterbesteck 31, 33 mit doppelter Länge der Seele verwendet werden. Durch die parallele Anordnung des Hauptkanals 38 und des Schnittes 42 des Seitenkanals 39 wird die Handhabung des Katheterbesteckes erleichtert und verbessert.

**Patentansprüche**

1. Katheterbesteck, bestehend aus einem Führungsstück (1; 37) mit einem Kanal (5; 39) zum Durchlass eines Katheterschlauches (23; 31), in dessen Lumen sich eine drahtförmige, flexible Seele (24; 33) mit stumpfer Spitze erstreckt, wobei die Seele und der Katheterschlauch an ihrem patientenfernen Ende miteinander verbindbare Kupplungsteile (25, 26; 30, 32) tragen und an dem patientenfernen Ende des Kanals ein Schutzschlauch (21) für den Katheterschlauch und die Seele befestigt ist, dadurch gekennzeichnet, dass das patientennahe Ende der Seele (24; 33) über die Spitze (28; 35) des Katheterschlauches (23; 31) ein Stück (27; 34) übersteht und dass das überstehende Stück (27; 34) der Seele (24; 33) sich in dem Kanal (5; 39) des Führungsstückes (1; 37) befindet.

2. Katheterbesteck nach Anspruch 1, dadurch gekennzeichnet, dass das überstehende Stück (27; 34) der Seele (24; 33) weich und flexibel ausgebildet, etwa halbkreisförmig gekrümmt und etwa so lang wie der Krümmungsabschnitt ist.

3. Katheterbesteck nach Anspruch 1 oder 2, dadurch gekennzeichnet, dass das überstehende Stück (27; 34) der Seele (24; 33) etwa 1,5 bis 3 cm lang ist.

4. Katheterbesteck nach einem der Ansprüche 1 bis 3, dadurch gekennzeichnet, dass die Seele (24) um das über die Spitze (28) des Katheterschlauches (23) überstehende Stück (27) länger ist als der Katheterschlauch (23).

5. Katheterbesteck nach einem der Ansprüche 1 bis 3, dadurch gekennzeichnet, dass die Seele (33) mit dem über die Spitze (35) des Katheterschlauches (31) überstehenden Stück (34) etwa doppelt so lang ist wie der Katheterschlauch (31).

6. Katheterbesteck nach einem der Ansprüche 1 bis 5, dadurch gekennzeichnet, dass der Kanal (5; 39) des Führungsstückes (1; 37) länger ist als das über die Spitze (28; 35) des Katheterschlauches (23; 31) überstehende Stück (27; 34) der Seele (24; 33).

**Revendications**

1. Instrument de cathétérisme, comportant une pièce de guidage (1; 37) pourvue d'un canal (5; 39) pour le passage d'un tube cathéter (23; 31) à l'intérieur duquel s'étend une âme flexible (24; 33) filiforme, pourvue d'une pointe emoussée; l'âme et le tube cathéter portant à leur extrémité située à l'opposé du patient des pièces d'accouplement (25, 26; 30, 32) pouvant être engagées l'une dans l'autre; et un tube de protection (21), pour le tube cathéter et l'âme, étant fixé à l'extrémité du canal qui est située à l'opposé du patient; caractérisé en ce qu'une partie (27; 34) de l'extrémité de l'âme (24; 33) proche du patient est en saillie au-delà de la pointe (28; 35) du tube cathéter (23; 31), et en ce que cette partie en saillie (27; 34) de l'âme (24; 33) se trouve dans le canal (5; 39) de la pièce de guidage (1; 37).

2. Instrument de cathétérisme selon la revendication 1, caractérisé en ce que la partie en saillie (27; 34) de l'âme (24; 33) est réalisée de manièer à être souple et flexible, à se recourber sensiblement en demi-cercle, et à s'étendre environ sur toute la longueur de ladite partie en saillie.

3. Instrument de cathétérisme selon l'une des revendications 1 ou 2, caractérisé en ce que la partie en saillie (27; 34) de l'âme (24; 33) a une longueur d'environ 1,5 à 3 cm.

4. Instrument de cathétérisme selon l'une des revendications 1 à 3, caractérisé en ce que l'âme (24) présente par rapport au tube cathéter (23) un excédent de longueur qui correspond à la partie (27) de l'âme qui est en saillie au-delà de la pointe (28) du tube cathéter (23).

5. Instrument de cathétérisme selon l'une des revendications 1 à 3, caractérisé en ce que l'âme (33), y compris sa partie (34) en saillie au-delà de la pointe (35) du tube cathéter (31), a sensiblement le double de la longueur du tube cathéter (31).

6. Instrument de cathétérisme selon l'une des revendications 1 à 5, caractérisé en ce que le canal (5; 39) de la pièce de guidage (1; 37) est plus long que la partie (27; 34) de l'âme (24; 33) qui est en saillie au-delà de la pointe (28; 35) du tube cathéter (23; 31).

**Claims**

1. A catheter instrument, comprising a guiding part (1; 37) having a channel (5; 39) for the passage of a catheter tube (23; 31), in the lumen of which extends a wire-form, flexible core (24; 33) having a blunt tip, the core and the catheter tube having coupling parts (25, 26; 30, 32) at the end thereof remote from the patient which are connectable with each other and a protective tube (21) for the catheter tube and the core is fixed at the end of the channel remote from the patient, characterised in that at the end of the core (24; 33) near the patien a part (27; 34) projects beyond the tip (28; 35) of the catheter tube (23; 31) and that the projecting part (27; 34) of the core (24; 33) is located in the channel (5; 39) of the guiding part (1; 37).

2. A catheter instrument according to claim 1, characterised in that the projecting part (27; 34) of the core (24; 33) is soft and flexible, is curved in an approximately semi-circular shape and is approximately as long as the curved section.

3. A catheter instrument according to claim 1 or 2, characterised in that the projecting part (27; 34) of the core (24; 33) is from about 1.5 to 3 cm long.

4. A catheter instrument according to one of claims 1 to 3, characterised in that the core (24) is longer than the catheter tube (23) by the length of the part (27) which projects beyond the tip (28) of the catheter tube (23).

5. A catheter instrument according to one of claims 1 to 3, characterised in that the core (33) with the part (34) which projects beyond the tip (35) of the catheter tube (31) is about twice as long as the catheter tube (31).

6. A catheter instrument according to to one of claims 1 to 5, characterised in that the channel (5; 39) of the guiding part (1; 37) is longer than the part (27; 34) of the core (24; 33) which projects beyond the tip (28; 35) of the catheter tube (23; 31).

FIG.1

FIG. 3

FIG. 2

FIG.4

FIG.5